# EUROPÄISCHE PATENTSCHRIFT

(11) **EP 0 998 683 B1**
(45) Veröffentlichungstag und Bekanntmachung des Hinweises auf die Patenterteilung: **23.05.2001**
(21) Anmeldenummer: 98940237.5
(22) Anmeldetag: 16.07.1998
(51) Int. Cl.: G01S 7/52, A61B 5/0456, G01S 15/89

(54) **VERFAHREN ZUR AUFNAHME VON ULTRASCHALLBILDERN BEWEGTER OBJEKTE**
METHOD FOR RECORDING ULTRASOUND IMAGES OF MOVING OBJECTS
PROCEDE D'ENREGISTREMENT D'IMAGES ULTRASONORES D'OBJETS ANIMES

(30) Priorität: 25.07.1997 DE 19732125
(43) Veröffentlichungstag der Anmeldung: 10.05.2000
(73) Patentinhaber: TomTec Imaging Systems GmbH, 85716 Unterschleissheim (DE)
(72) Erfinder: MUMM, Bernhard, D-82291 Mammendorf (DE); WALDINGER, Johannes, D-85579 Neubiberg (DE); KAISER, Dietmar, D-85368 Moosburg (DE)
(74) Vertreter: Müller, Frank Peter, Dipl.-Ing.
(86) Internationale Anmeldenummer: EP9804430
(87) Internationale Veröffentlichungsnummer: WO9905542

(56) Entgegenhaltungen:
- EP-A- 0 736 284
- EP-A- 0 802 423
- US-A- 5 315 512
- MCCANN H A ET AL: "MULTIDIMENSIONAL ULTRASONIC IMAGING FOR CARDIOLOGY" PROCEEDINGS OF THE IEEE, Bd. 76, Nr. 9, 1. September 1988, Seiten 1063-1072, XP000112039

## Beschreibung

Die Erfindung betrifft ein Verfahren zur Aufnahme von Ultraschallbildern bewegter Objekte, insbesondere von Blutgefäßen oder Organen von Lebewesen, nach dem Oberbegriff des Anspruches 1.

Zur Aufnahme von Objekten mittels Ultraschallgeräten wird ein Ultraschallsender zur Abstrahlung von Ultraschallwellen auf das Objekt gerichtet, während ein Ultraschallempfänger die von dem Objekt reflektierten Ultraschallwellen empfängt. Dafür werden üblicherweise Ultraschallköpfe verwendet, die sowohl den Ultraschallsender als auch den Ultraschallempfänger beinhalten. Diese Ultraschallköpfe werden zur Aufnahme einer Vielzahl von zweidimensionalen Bildern entlang dem zu untersuchenden Objekt bewegt, während eine Vielzahl von einzelnen Bildern des Objekts erstellt werden. Die Bewegung des Ultraschallkopfes kann dabei linear, zirkular, bogenförmig oder in einer beliebigen anderen Richtung, beispielsweise freihand, erfolgen. Diese Bilder stellen einzelne Bild-Teilbereiche des Objekts dar und werden in einem Datenverarbeitungssystem zusammengesetzt, so daß sich ein dreidimensionales Volumenbild ergibt. Das dreidimensionale Volumenbild besteht dabei aus einer "Übereinanderschichtung" der einzelnen Bild-Teilbereiche, die jeweils einzelne "Schichten" des zu untersuchenden Objekts darstellen. Durch die Bewegung des Ultraschallkopfes entlang dem zu untersuchenden Objekt wird dieses schichtweise gescannt, wobei bei einer linearen Abtastung die Anzahl der Schichten pro Längeneinheit die Auflösung des resultierenden Ultraschallbildes bestimmen. Bei zirkularen oder anderen Abtastbewegungen werden die Ultraschallbilder entsprechend der inkrementalen Aufnahme von einzelnen Bild-Teilbereichen zusammengesetzt und dargestellt.
Die Bewegung des Schallkopfes kann durch eine Mechanik, durch eine in den Schallkopf integrierte Schallablenkeinrichtung oder durch Freihandbildaufnahmen mit magnetischen oder optischen Sensorsystemen zur dreidimensionalen Zuordnung der Ultraschallbilder realisiert werden. Die Zuordnung der einzelnen "Schichten" des Objekts kann beispielsweise auch durch das Verfahren der vom Anmelder getätigten parallelen Patentanmeldung bewerkstelligt werden. Die Zusammenstellung der Ultraschallbilder zur Erfassung der dritten Dimension erfolgt üblicherweise in einem Datenverarbeitungssystem, welches das Videoausgangssignal oder ein digitales Ausgangssignal des Ultraschallsystems erfaßt und entsprechend auswertet. Die entsprechenden Signale liefert der Ultraschallkopf, respektive der Ultraschallempfänger.
Die Bilder von Objekten, die durch ihre Eigenbewegung oder durch die Bewegung benachbarter Objekte eine Bewegungsunschärfe erzeugen, werden üblicherweise auf diese Bewegung synchronisiert aufgenommen. Ohne die Synchronisierung auf die entsprechende Bewegung des Objekts (stroposkopartige Aufnahme) ergibt sich ein unscharfes Bild bzw. eine Darstellung des Objekts in jedem seiner Bewegungszustände. Bei einer auf die Bewegung des Objekts synchronisierten Aufnahme entsteht je Bewegungszustand des jeweiligen Objekts ein Volumenbild. Ein Beispiel dafür ist die dreidimensionale Ultraschallbildaufnahme des Herzens. Die entsprechenden Volumenbilder des Herzens zeigen nacheinander alle Stufen (Phasen) zwischen Kontraktion (Systole) und Erschlaffung des Herzens (Diastole). Die sukzessive Darstellung einzelner Volumenbilder des Herzens entspricht einer vierdimensionalen Darstellung des Herzens, wobei die vierte Dimension die entsprechende Bewegung des Herzens repräsentiert.

Zur Diagnose der einzelnen Zustände des zu untersuchenden Objektes, zu bestimmten Zeitpunkten der entsprechenden Bewegung des Objekts, ist jeweils nur ein Zustand des Objekts wichtig. Beispielsweise ist zu bestimmten Zeitpunkten des Elektrokardiogramms oder der Atmung, der Bewegung des Magens oder der Perestaltik der Speiseröhre eines Patienten der jeweilige Organzustand für eine gezielte Diagnose wichtig. Beispielsweise wird die Stenose (Engstelle) eines Blutgefäßes zum Zeitpunkt der Diastole betrachtet (vgl. Prospekt "Technology Making A Difference" der Firma TomTec Imaging Systems GmbH, August 1996).

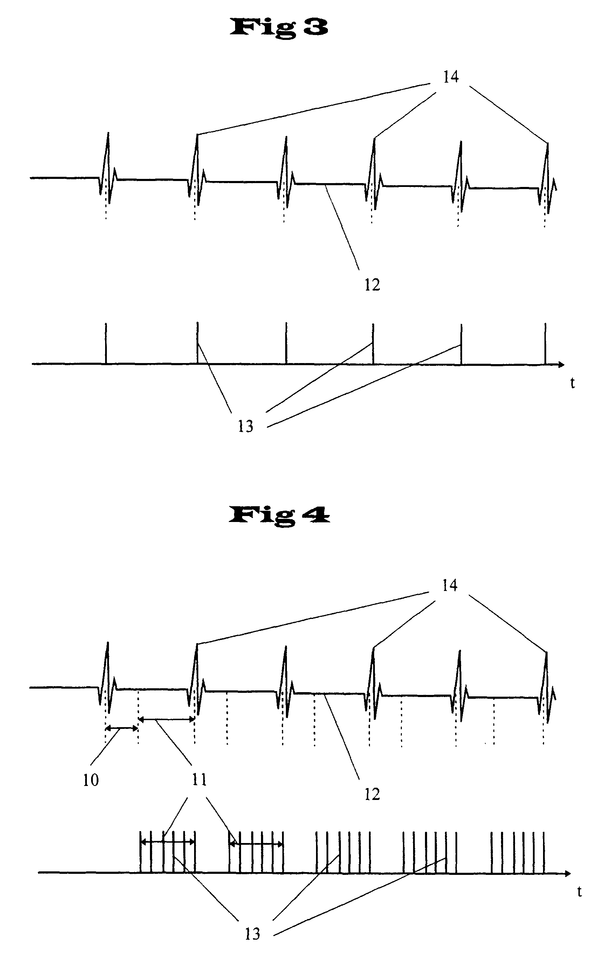

Aus der EP 0 736 284 A2 ist ein Verfahren bekannt, bei dem bei der Aufnahme von Blutgefäßen oder Organen je Bewegungszyklus, z.B. Systole-Diastole-Zyklus (RR-Intervall) eine einzige Schicht aufgenommen wird (vgl. auch Figur 3). Die erreichbare Bildaufnahmegeschwindigkeit ist dabei sehr gering. Für Aufnahmen, die der Herzbewegung folgen, d.h. die der Pulsfrequenz des Patienten folgen, wird pro Sekunde ca. eine Schicht aufgenommen, sofern die Pulsfrequenz etwa 60 Herzschläge pro Minute zeigt. Die erreichbare Bildqualität ist gut, die langen Aufnahmezeiten sind in der Routine in den meisten Fällen jedoch nicht akzeptabel.

Bei der Bildaufnahme von Herzkranzgefäßen mittels Katheter ist die Zeitdauer der Untersuchung sehr kurz zu halten, da der Katheter das Gefäß ganz oder teilweise verschließt, welches ein Risikofaktor für den Patienten darstellt. Hier sind die oben erwähnten langen Aufnahmezeiten für den Patienten eine große Belastung.

Bewegungen des Patienten, die zu Bewegungsartefakten im drei- oder vier- dimensionalen Bild führen, lassen sich über den langen Zeitraum der Bildaufnahme, z.B. bei der Karotis (Schlucken, Niesen, Husten, Kopf- oder Halsbewegung, etc...) nicht ausschließen. Artefakte, d.h. Unschärfen im Ultraschallbild (die räumlich falsche Zuordnung einzelner den Bild-Teilbereichen entsprechender Ultraschallbilder durch Verschiebung des Organs oder eines Teils des Organs oder der Zuordnung Organ-Ultraschallkopf), sind dann die Folge. Als Ausweg benutzen herkömmliche Verfahren sehr große Schrittweiten pro Schicht oder es wird mit einer sehr schnellen Schallkopfbewegung längs des zu untersuchenden Organs gearbeitet. Dies führt zu einer inhomogenen Auflösung im dreidimensionalen Volumenbild in Richtung der Bewegung des Schallkopfes.

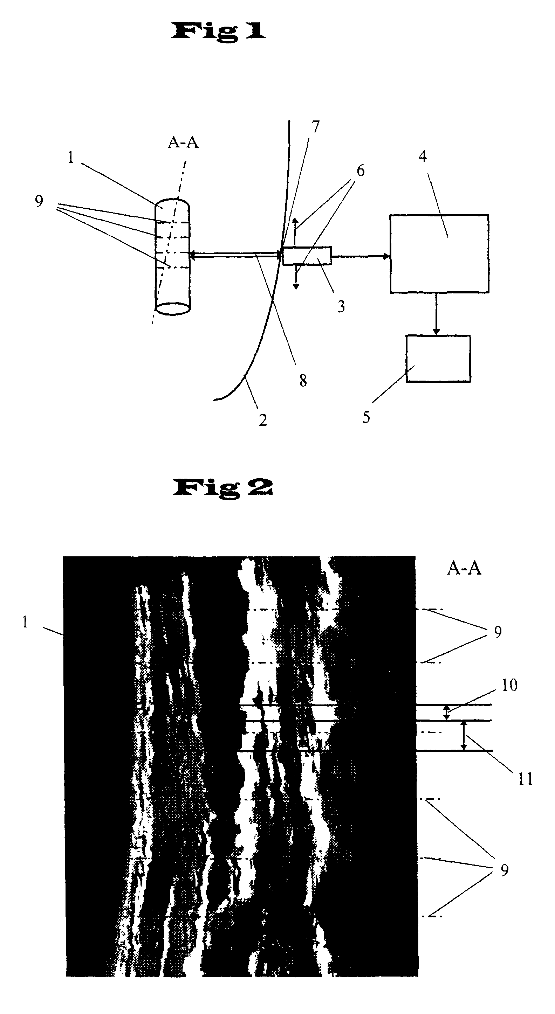

Bei einer kontinuierlichen Bildaufnahme während der Bewegung des Schallkopfes und bei Kopplung des Schallkopfes an die Videonorm, z.B. PAL, werden pro Sekunde in etwa 25 Schichten aufgenommen. Das dreidimensionale Bild wird jedoch durch die Bewegung des Organs oder des Blutgefäßes während der Systole und während der Diastole stark beeinträchtigt. Die Bilder könnnen zur anschließenden Auswertung nur eingeschränkt verwendet werden und eine Fehlinterpretation der Bildinhalte durch den Arzt ist möglich (vgl. Fig.2).

Der Erfindung liegt die Aufgabe zugrunde, die Bewegungsunschärfe bei der Aufnahme von Ultraschallbildern eines bewegten Objekts zu minimieren und gleichzeitig die Bildaufnahmezeit zu verkürzen. Dabei soll das Verfahren einfach handhabbar gestaltet sein und sich mit herkömmlichen Ultraschallgeräten und Datenverarbeitungssystemen realisieren lassen.

Die Aufgabe der Erfindung wird durch die kennzeichnenden Merkmale des Patentanspruches 1 gelöst. Besondere Ausführungsformen der Erfindung sind in den Unteransprüchen gekennzeichnet.

Während der Ultraschallkopf zur Aufnahme von einzelnen Bild-Teilbereichen des bewegten Objekts entlang dem Objekt verfahren wird und dabei zu bestimmten Zeitpunkten Aufnahmen des Objekts erstellt, die die einzelnen "Schichten" des Objekts darstellen und die später in dem Datenverarbeitungssystem zu einem dreidimensionalen Volumenbild zusammengesetzt werden, werden zu den Aufnahmen der einzelnen Bild-Teilbereiche die Bewegungszustände des Objekts erfaßt. Die Aufnahmen der einzelnen Bild-Teilbereiche werden dann den Bewegungszuständen des Objekts zugeordnet und entsprechend den Bewegungszuständen des Objekts zusammengesetzt und dargestellt.

Insbesondere werden die Zeitpunkte zur Aufnahme von einzelnen Bild-Teilbereichen des Objekts durch die Bewegungsabläufe des Objekts gesteuert. Beispielsweise werden die Zeitpunkte zur Aufnahme der einzelnen Bild-Teilbereiche des bewegten Objekts derart festgelegt oder geregelt, daß während der größten Bewegung des Objekts keine oder nur wenige Aufnahmen und zu den Zeitpunkten geringerer Bewegung des Objekts mehrere Aufnahmen erstellt werden.

Zur Aufnahme von Blutgefäßen oder Organen eines Lebewesens werden während des systolischen Zyklus keine oder nur wenige Aufnahmen und während des diastolischen Zyklus mehrere Aufnahmen von einzelnen Bild-Teilbereichen des Blutgefäßes oder des Organs erstellt. Ist die Bewegung des zu untersuchenden Objekts bekannt, so können die einzelnen Bild-Teilbereiche auch zu diskreten, d.h. fest definierten Zeitpunkten aufgenommen werden. Zur Aufnahme von durch den Herzschlag bewegten Objekten innerhalb eines Lebewesens eignet sich die Steuerung der Zeitpunkte der Aufnahme von einzelnen Bild-Teilbereichen durch die Signale des Elektrokardiogramms des Lebewesens.

Während der Ultraschallkopf längs des Organs des Lebewesens verfahren wird, werden kontinuierlich einzelne Aufnahmen unterschiedlicher Bild-Teilbereiche des Organs aufgenommen. Durch Kopplung der Aufnahmezeiten mit dem EKG des Lebewesens wird die Systole ausgelassen, d.h. es werden beispielsweise für einen Zeitraum von etwa 200 ms (bei einer Pulsfrequenz von 60) keine Aufnahmen erstellt, um anschließend etwa alle 120 bis 200 ms entsprechend dem Elektrokardiogramm während der Diastole mehrere Aufnahmen zu erstellen. Dies ist auch durch eine kontinuierliche Aufnahme des Organs möglich, wobei das Datenverarbeitungssystem die entsprechenden Aufnahmen ausselektiert und die gewünschten Aufnahmen verarbeitet. In der Zeit der größten Bewegungsartefakte durch die Pulsation werden also die entsprechenden Aufnahmen ausgelassen, während danach bei einer Aufnahmegeschwindigkeit von z.B. 25 Schichten pro Sekunde, z.B. jedes fünfte Bild aufgenommen wird. Die Scanbewegung des Ultraschallkopfes erfolgt dabei kontinuierlich. Dadurch erreicht man eine annähernd homogene Ortsauflösung für den Scan von ca. vier Schichten pro Sekunde.

Das Verfahren ist besonders im IVUS-Bereich einsetzbar. Hier sind lange Aufnahmezeiten im Interesse des Patienten unakzeptabel (z.B. 30 mm mit 0,1 mm Inkrement bei Puls 60 Schlägen pro Minute: dies entspricht in etwa 300 Schichten und ergibt eine Gesamtaufnahmezeit von etwa 5 Minuten).

Mit dem erfindungsgemäßen Verfahren läßt sich die Aufnahmezeit auf ca. 25 % der herkömmlichen Aufnahmezeiten (bei EKG-Triggerung) verringern. Vorteilhafterweise werden während des systolischen Zyklus keine Aufnahmen und während des diastolischen Zyklus etwa 2 bis 20, insbesondere 2 bis 10 Aufnahmen von einzelnen Bild-Teilbereichen des bewegten Organs verwertet. Insbesondere eignet sich das Verfahren für Flußdarstellungen, d.h. durch farblich gekennzeichnete Darstellungen des bewegten Objekts, bzw. einzelner Bereiche des Objekts.

Das Verfahren läßt sich auch dadurch realisieren, daß der Ultraschallkopf während der Bewegung entlang dem zu untersuchenden Objekts kontinuierlich einzelne Bild-Teilbereiche des Objekts aufnimmt und anschließend ein Datenverarbeitungssystem die Aufnahmen der Zeitpunkte größter Bewegung des Objekts ausfiltert. Diese Ausfilterung ist auch bei einem Aufnahmeverfahren zweckmäßig, bei dem die Zeitpunkte zur Aufnahme von einzelnen Bild-Teilbereichen durch das Elektrokardiogramm des Lebewesens gesteuert werden, und zwar dann, wenn beispielsweise auch während der Diastole Extrasystolen, d.h. außerordentliche Bewegungen des Organs bzw. des zu untersuchenden Objekts auftreten und diese Bildaufnahmen dann ausgefiltert werden können. Durch gezielte Filterung lassen sich auch brauchbare Bilder von Patienten erstellen, die unter einer Arhytmie leiden, wobei hier gewährleistet sein muß, daß noch eine genügende Anzahl von Bildern während der erschlafften Phasen des Herzens erstellt werden können.

Werden die Bild-Teilbereiche eines Objekts kontinuierlich aufgenommen, so besteht die Möglichkeit, jeweils die den einzelnen Bewegungszuständen des Objekts zugehörigen Ultraschallbilder zu einem Zustandsbild des Objekts zusammenzufassen. Jeweils eine Phase des Objekts ergibt ein entsprechendes Volumenbild durch Zusammenstellung einer Vielzahl von Ultraschallbildern die zu einem bestimmten Bewegungszustand aufgenommen wurden. Die sukzessive Aneinanderreihung dieser Volumenbilder ergibt eine Darstellung des Bewegungsablaufes des Objekts in Echtzeit, vergleichbar mit einem "Film". Eine sog. vierdimensionale Darstellung des bewegten Objekts trotz geringer Aufnahmezeiten ist möglich.

Ein besonderes Ausführungsbeispiel wird anhand der Zeichnungen wie folgt erläutert. Dabei zeigen:
- Figur 1: einen Schnitt durch ein Ultraschallsystem (Ultraschallbild-Untersuchungsanordnung), zur Untersuchung der Halsschlagader eines Patienten;
- Figur 2: ein sich ergebendes Ultraschallbild bei unsynchronisierter, d.h. kontinuierlicher Aufnahme der Halsschlagader nach Figur 1 durch ein herkömmliches Verfahren;
- Figur 3: die schematische Darstellung des Herzschlages (z.B. EKG) sowie der diskrete Aufnahmezeitpunkte herkömmlicher Verfahren;
- Figur 4: die schematische Darstellung des Herzschlages (z.B. EKG) nach Figur 3 sowie die erfindungsgemäßen Aufnahmezeitpunkte während der Diastole; und
- Figur 5: die schematische Darstellung des Herzschlages (z.B. EKG) nach Figur 3 sowie die erfindungsgemäßen Aufnahmezeitpunkte einzelner Bewegungszustände und deren Zuordnung.

Figur 1 zeigt die schematische Darstellung der Ultraschallbild-Untersuchungsanordnung zur Untersuchung der Halsschlagader, d.h. zur Untersuchung eines bewegten Objekts 1. Ein Ultraschallkopf 3 wird längs der Verfahrrichtung 6 entlang dem zu untersuchenden Objekt 1 verfahren, während verschiedene "Schichten" d.h. Bild-Teilbereiche 9 des Objekts 1 aufgenommen werden. Zwischen dem Ultraschallkopf 3 und der Körperwand 2 befindet sich üblicherweise ein Schallmedium 7, wie beispielsweise Öl, Gel oder Wasser, welches die Ultraschallwellen 8 vom Ultraschallkopf 3 zur Körperwand 2 leitet. Der Ultraschallkopf 3 liefert die entsprechenden Bildsignale an ein Datenverarbeitungssystem 4, welches nach der Verarbeitung der Ultraschallbilder ein dreidimensionales oder vierdimensionales (d.h. ein bewegtes dreidimensionales Bild) an ein Anzeigegerät 5 weiterleitet.

Die einzelnen Bild-Teilbereiche 9 werden in dem Datenverarbeitungssystem 4 zusammengesetzt, so daß sich am Anzeigegerät 5 ein drei- oder vierdimensionales Bild ergibt. Die Ansteuerung des Ultraschallkopfes 3 sowie die Bewegung des Ultraschallkopfes 3 übernimmt entweder das Datenverarbeitungssystem 4 oder ein hier nicht dargestelltes Steuergerät.

Figur 2 zeigt den Schnitt A-A nach Figur 1 durch eine Halsschlagader eines zu untersuchenden Patienten, wobei das hier gezeigte Ultraschallbild durch die kontinuierliche Aufnahme einzelner Bild-Teilbereiche 9 entstanden ist.

In dem Ultraschallbild nach Figur 2 erkennt man eine als Wellenlinie sich darstellende Halsschlagaderwand, die dadurch entstanden ist, daß sich während des Scanvorganges (ca. 25 Bilder pro Sekunde) durch den Herzschlag des Patienten die Halsschlagader bewegt, d.h. jeweils größere bzw. kleinere Durchmesser aufweist und sich diese sich ändernden Durchmesser in einem Bild dargestellt werden. So ergibt sich während der Systole und während der Diastole des Herzens ein kleinerer bzw. größerer Durchmesser bzw. eine Lageänderung der Halsschlagader. In dem Ultraschallbild nach Figur 2 erkennt man in etwa 8 Pulsschläge des Herzens, wobei sich jeder Pulsschlag in einen systolischen Zyklus 10 und einen diastolischen Zyklus 11 aufteilt. Die einzelnen Bild-Teilbereiche 9 sind schematisch dargestellt, wobei das hier gezeigte Ultraschallbild aus etwa 200 Bild-Teilbereichen 9 aufgebaut wurde.

Figur 3 zeigt schematisch das EKG 12, das den Bewegungsablauf des Herzens (den Herzschlag eines Patienten) repräsentiert und das in gleichmäßigen Abständen Bewegungsmaxima 14 aufweist, während zwischen den Bewegungsmaxima, d.h. während der Erschlaffung des Herzens, erfahrungsgemäß Zeiten geringerer Bewegung vorzufinden sind.

Im unteren Teil der Figur 3 erkennt man die Aufnahmezeitpunkte 13, die entsprechend den Bewegungsmaxima 14 des Herzens angeordnet sind, so daß sich durch dieses Aufnahmeverfahren stroposkopartig die Bewegungszustände der durch den Herzschlag bewegten Organe jeweils vor Beginn des systolischen Zyklus10 darstellen läßt. Eine zeitliche Verschiebung der Aufnahmezeitpunkte durch ein Zeit-Offset ist üblicherweise möglich.

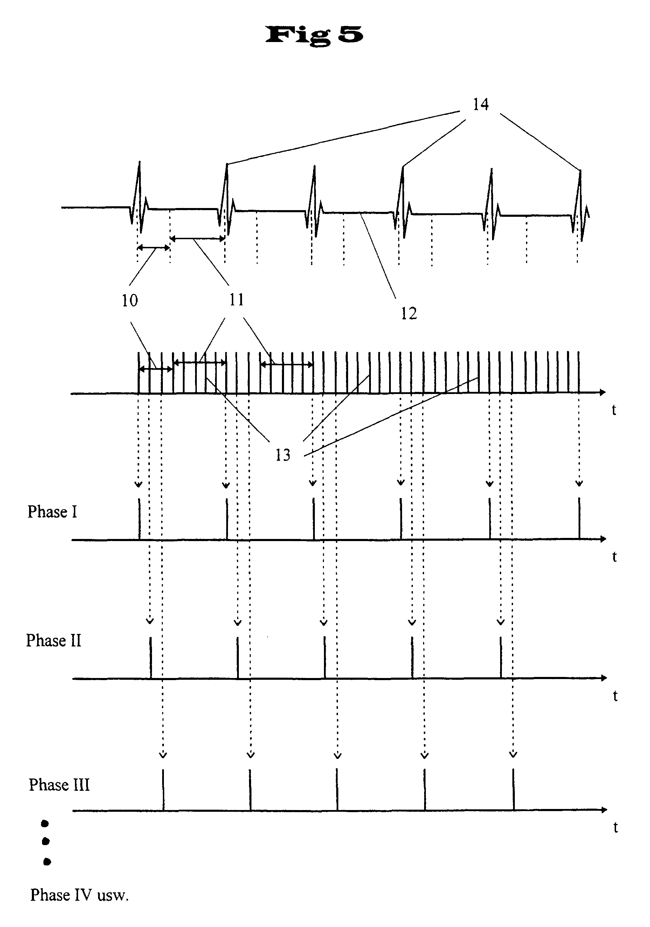

Figur 4 zeigt ebenfalls schematisch das EKG 12 eines Patienten mit den Bewegungsmaxima 14. Nach dem erfindungsgemäßen Verfahren werden hier eine Anzahl von unterschiedlichen Aufnahmezeitpunkten 13 definiert, die während des diastolischen Zyklus 11 aufgenommen werden. Während des systolischen Zyklus 10, d.h. während der größten Bewegung des Herzens, werden keine Aufnahmen erstellt, um eine Bewegungsunschärfe des dreidimensionalen Ultraschallbildes zu vermeiden. Durch dieses Verfahren läßt sich das jeweils zu untersuchende Organ, welches durch den Herzschlag des Patienten bewegt wird, während des diastolischen Zyklus 11 darstellen, wobei sich hier aufgrund einer Mehrzahl von Aufnahmen von Bild-Teilbereichen 9 während des diastolischen Zyklus 11 die Bewegungsunschärfe des Ultraschallbildes minimieren läßt. Die Bildaufnahmezeit verkürzt sich gleichzeitig um ein Vielfaches.

Figur 5 zeigt die schematische Darstellung des EKGs 12 nach Figur 3 sowie die erfindungsgemäßen Aufnahmezeitpunkte einzelner Bewegungszustände und deren Zuordnung. Die Bild-Teilbereiche 9 werden kontinuierlich aufgenommen. Durch Zuordnung einzelner Aufnahmezeitpunkte, d.h. der entsprechenden Bild-Teilbereiche 9, zu den jeweiligen Bewegungszuständen des Objekts 1 ergeben sich die entsprechenden dreidimensionalen Volumenbilder einzelner Bewegungszustände des Objekts. Je nach Phase, d.h. je nach Bewegungszustand des Objekts 1 werden einzelne Bild-Teilbereiche 9 zusammengestellt ("aufeinandergeschichtet") und ergeben in der Darstellung ein dreidimensionales Bild des Objekts 1 zu einem bestimmten Bewegungszustand. Die Aneinanderreihung dieser einzelnen Volumenbilder ergibt eine Echtzeit-Darstellung der Bewegung des Objekts wie in einem Film. Auch schnellere oder langsamere (Zeitlupe) Darstellungen der Bewegungen des Objekts lassen sich dadurch realisieren. Die Selektion der einzelnen Aufnahmezeitpunkte übernimmt das Datenverarbeitungssystem, welches auch die Zuordnung der einzelnen Bild-Teilbereiche zu einem Volumenbild vornimmt.

## Patentansprüche

1. Verfahren zur Aufnahme von Ultraschallbildern eines bewegten Objekts, insbesondere eines Blutgefäßes,
mit einem Ultraschallsender zur Abstrahlung von Ultraschallwellen auf das Objekt, und einem Ultraschallempfänger zum Empfangen von von dem Objekt reflektierten Ultraschallwellen,
wobei der Ultraschallsender und/oder der Ultraschallempfänger entlang dem Objekt verfahren wird und entsprechend den Bewegungen des Objekts zu bestimmten Zeitpunkten Aufnahmen von einzelnen Bild-Teilbereichen des Objekts erstellt werden und zu den Aufnahmen der einzelnen Bild-Teilbereiche (9) die Bewegungszustände des Objekts (1) erfaßt und den einzelnen Bild-Teilbereichen (9) zugeordnet werden, und dann die Aufnahmen der einzelnen Bild-Teilbereiche (9) entsprechend den Bewegungszuständen des Objekts (1) zusammengesetzt und dargestellt werden,
**dadurch gekennzeichnet,**
daß mehrere Bild-Teilbereiche (9) für denselben Bewegungszustand des Objekts (1) entweder kontinuierlich aufgenommen werden und ein Datenverarbeitungssystem (4) die Aufnahmen der Zeitpunkte größter Bewegung des Objekts (1) selektiert, oder
daß zu den Zeitpunkten größter Bewegung des Objekts (1) keine oder nur wenige Aufnahmen und zu den Zeitpunkten geringerer Bewegung des Objekts mehrere Aufnahmen von einzelnen Bild-Teilbereichen (9) für denselben Bewegungszustand des Objekts (1) erstellt werden.

2. Verfahren nach Anspruch 1,
**dadurch gekennzeichnet,**
daß die Zeitpunkte zur Aufnahme von einzelnen Bild-Teilbereichen (9) des Objekts (1) durch die Bewegungsabläufe des Objekts (1) gesteuert werden.

3. Verfahren nach Anspruch 2,
**dadurch gekennzeichnet,**
daß die Zeitpunkte für die Aufnahme von einzelnen Bild-Teilbereichen (9) des Objekts (1) durch Signale des Elektrokardiogramms, der Atmung, der Magenbewegung, der Perestaltik der Speiseröhre oder einer Kombination aus diesen Signalen eines Lebewesens gesteuert werden.

4. Verfahren nach einem der vorhergehenden Ansprüchen,
**dadurch gekennzeichnet,**
daß zur Aufnahme von Blutgefäßen oder Organen eines Lebewesens während des systolischen Zyklus (10) keine oder nur wenige Aufnahmen und während des diastolischen Zyklus (11) mehrere Aufnahmen von einzelnen Bild-Teilbereichen (9) des Blutgefäßes oder des Organs erstellt werden.

5. Verfahren nach einem der vorhergehenden Ansprüche,
dadurch gekennzeichnet,
daß die einzelnen Bild-Teilbereiche (9) des Blutgefäßes oder des Organs zu diskreten, fest definierten Zeitpunkten aufgenommen werden.

6. Verfahren nach einem der vorhergehenden Ansprüche,
**dadurch gekennzeichnet,**
daß während des systolischen Zyklus (10) keine Aufnahmen und während des diastolischen Zyklus (11) etwa 2 bis 10 Aufnahmen von einzelnen Bild-Teilbereichen (9) des Blutgefäßes oder des Organs erstellt werden.

7. Verfahren nach einem der vorhergehenden Ansprüche,
**dadurch gekennzeichnet,**
daß Aufnahmen einzelner Bild-Teilbereiche (9) entsprechend sich wiederholender Bewegungszustände des Objekts (1) zu einem Zustandsbild des Objekts (1) zusammengefaßt und dargestellt werden.

8. Verfahren nach Anspruch 7,
**dadurch gekennzeichnet,**
daß die Zustandsbilder einzelner Bewegungszustände des Objekts (1) sukzessive entsprechend dem Bewegungsablauf des Objekts (1) dargestellt werden.

9. Verfahren nach einem der vorhergehenden Ansprüche,
**dadurch gekennzeichnet,**
daß die Zeitpunkte zur Aufnahme von einzelnen Bild-Teilbereichen (9) des Objekts (1) anhand von Signalen des Elektrokardiogramms des Lebewesens gesteuert werden und
daß durch ein Datenverarbeitungssystem (4) einzelne Aufnahmen der Zeitpunkte größter Bewegung des Objekts (1) ausgefiltert werden.

10. Verfahren nach einem der vorhergehenden Ansprüche,
**dadurch gekennzeichnet,**
daß der Ultraschallsender und/oder der Ultraschallempfänger linear, zirkular, bogenförmig oder freihand entlang dem Objekt (1) verfahren wird.

11. Verfahren nach einem der vorhergehenden Ansprüche,
**dadurch gekennzeichnet,**
daß einzelne Bereiche des Objekts (1) insbesondere unterschiedlich bewegte Bereiche des Objekts (1), farblich gekennzeichnet werden.

## Claims

1. Method of recording ultrasonic images of a moving object, particularly a blood vessel, by means of an ultrasonic emitter for emission of ultrasonic waves onto said object, and an ultrasonic receiver for receiving ultrasonic waves reflected by said object, wherein said ultrasonic emitter and/or said ultrasonic receiver is/are displaced along said object and individual image sub-areas (9) of said object are recorded at specified points of time, and wherein the states of motion of said object are detected for the images of said individual image sub-areas (9) and wherein then the images of the individual image sub-areas (9) are composed and displayed in correspondence with the states of motion of said object (1),
**characterised in**
that either several image sub-areas (9) either recorded for the same state of motion of said object (1) continuously, with a data processing system (4) selecting the images taken by the points of time of maximum motion of said object (1), or
that by the points of time of maximum motion of said object (1) no or only a few images are produced while by the points of time of slight motion of said object several images of individual image sub-areas (9) are recorded for the same state of motion of said object (1).

2. Method according to Claim 1,
**characterised in**
that the points of time for recording individual image sub-areas (9) of said object (1) are controlled by the sequence of movements of said object (1).

3. Method according to Claim 2,
**characterised in**
that the points of time for recording individual image sub-areas (9) of said object are controlled by signals of the electrocardiogram, the respiration, the movements of the stomach, the peristalsis of the oesophagus or a combination of these signals from an organism.

4. Method according to any of the preceding Claims,
**characterised in**
that for recording of blood vessels or organs of an organism no or only a few images are recorded during the systolic cycle (10) while several images are recorded of individual image sub-areas (9) of said blood vessel or said organ during the diastolic cycle (11).

5. Method according to any of the preceding Claims,
**characterised in**
that said individual image sub-areas (9) of said blood vessel or said organ are recorded at discrete and invariably defined points of time.

6. Method according to any of the preceding Claims,
**characterised in**
that no images are recorded during the systolic cycle (10) and approximately 2 to 10 images of individual image sub-areas (9) of said blood vessel or said organ are recorded during the diastolic cycle.

7. Method according to any of the preceding Claims,
**characterised in**
that images of individual image sub-areas (9) are combined and displayed in correspondence with repetitive states of motion of said object (1) to form an image of the condition of said object (1).

8. Method according to Claim 7,
**characterised in**
that the images of state of individual states of motion of said object (1) are displayed in succession in correspondence with the sequence of motions of said object (1).

9. Method according to any of the preceding Claims,
**characterised in**
that the points of time of recording individual image sub-areas (9) of said object are controlled on the basis of signals of the electrocardiogram of the organism and that isolated images recorded by the points of time of maximum motion of said object (1) are filtered out by means of a data processing system (4).

10. Method according to any of the preceding Claims,
**characterised in**
that said ultrasonic emitter and/or said ultrasonic receiver is/are displaced along linear, circular, bow-shaped or free-hand lines along said object (1).

11. Method according to any of the preceding Claims,
**characterised in**
that individual areas of said object, particularly areas of said object (1) which move in different ways, are identified in colours.

## Revendications

1. Procédé d'enregistrement d'images ultrasonores d'un objet animé, et d'un vaisseau sanguin en particulier, moyennant un émetteur à ultrason à émettre des ondes ultrasonores sur ledit objet, et un récepteur à ultrason à recevoir des ondes ultrasonores réfléchies par ledit objet, dans lequel ledit émetteur à ultrason et/ou ledit récepteur à ultrason est/sont déplacé(s) le long ledit objet et des sous-régions d'image individuelles dudit objet sont enregistrées aux points de temps spécifiés, et dans lequel les états des mouvement dudit objet sont détectés pour les images enregistrées desdites sous-régions d'image individuelles (9) sont composées et affichées en correspondance avec les états de mouvement dudit objet (1),
**caractérisé en ce**
que plusieurs sous-régions d'image (9) sont enregistrée pour le même état de mouvement dudit objet (1) en continu, à un système de traitement de l'information (4) choisissant les images enregistrées aux points de temps d'un maximum de mouvement dudit objet (1), ou
en ce qu'aux points de temps d'un maximum de mouvement dudit objet (1) aucune image, ou seulement quelques-unes images, sont produites, pendant qu'aux points de temps d'un léger mouvement dudit objet plusieurs images des sous-régions d'image individuelles (9) sont enregistrées pour le même état de mouvement dudit objet (1).

2. Procédé selon la revendication 1,
**caractérisé en ce**
que les points de temps d'enregistrement des sous-régions d'image individuelles (9) dudit objet (1) sont commandés par la succession des mouvements dudit objet (1).

3. Procédé selon la revendication 2,
**caractérisé en ce**
que les points de temps d'enregistrement des sous-régions d'image individuelles (9) dudit objet sont commandés par des signaux d'un électrocardiogramme, de la respiration, des mouvements de l'estomac, du péristaltisme de l'oesophage, ou par une combinaison des ces signaux d'un organisme.

4. Procédé selon une quelconque des revendications précédentes,
**caractérisé en ce**
que pour l'enregistrement des vaisseaux sanguins ou des organes d'un organisme aucune ou seulement quelques images sont enregistrées au cours du cycle de systole (10), pendant que plusieurs images sont enregistrées pour des of sous-régions d'image individuelles (9) dudit vaisseau sanguin ou dudit organe au cours du cycle de diastole (11).

5. Procédé selon une quelconque des revendications précédentes,
**caractérisé en ce**
que lesdites sous-régions d'image individuelles (9) dudit vaisseau sanguin ou dudit organe sont enregistrées aux points de temps discrètes et spécifiés à demeure.

6. Procédé selon une quelconque des revendications précédentes,
**caractérisé en ce**
qu'aucune image est enregistrée au cours du cycle de systole (10), et 2 à 10 images environ des sous-régions d'image individuelles (9) dudit vaisseau sanguin ou dudit organe sont enregistrées au cours du cycle de diastole.

7. Procédé selon une quelconque des revendications précédentes,
**caractérisé en ce**
que des images des sous-régions d'image individuelles (9) sont combinées et affichées en correspondance avec des états de mouvement itératifs dudit objet (1) afin de former une image de l'état dudit objet (1).

8. Procédé selon la revendication 7,
**caractérisé en ce**
que les images d'état des états de mouvement individuels dudit objet (1) sont affichées en succession en correspondance avec la succession de mouvements dudit objet (1).

9. Procédé selon une quelconque des revendications précédentes,
**caractérisé en ce**
que les points de time d'enregistrement des sous-régions d'image individuelles (9) dudit objet sont commandés à la base de signaux de l'électrocardiogramme de l'organisme, et
en ce que des images individuelles enregistrées aux points de temps à un maximum de mouvement objet (1) sont extraites par filtrage moyennant un système de traitement de l'information (4).

10. Procédé selon une quelconque des revendications précédentes,
**caractérisé en ce**
que ledit émetteur à ultrason et/ou ledit récepteur à ultrason est/sont déplacé(s) le long ledit objet (1) selon des lignes linéaires, circulaires, arquées ou à main levée.

11. Procédé selon une quelconque des revendications précédentes,
**caractérisé en ce**
que des zones individuelles sur ledit objet, en particulier des zones dudit objet (1) qui subissent un mouvement de manières différentes, sont identifiées en couleurs.
